# EUROPEAN PATENT APPLICATION

(11) **EP 3 858 995 A2**
(43) Date of publication of application: **04.08.2021**
(21) Application number: 19902868.9
(22) Date of filing: 27.12.2019
(51) Int. Cl.: C12N 15/74, C12N 15/67, C07K 14/575, A23L 33/135, A61K 35/747, A61K 45/06, A61P 1/00, A61P 29/00

(54) **EXOGENOUS PROTEIN-EXPRESSING MICROORGANISM AND USE THEREOF**

(30) Priority: 28.12.2018 KR 20180173063
(71) Applicant: Medytox Inc., Chungcheongbuk-do 28126 (KR)
(72) Inventor: SONG, Ji Yoon, Seongnam-si, Gyeonggi-do 13479 (KR); NOH, Hyeon Jin, Suwon-si, Gyeonggi-do 16225 (KR); JUNG, Yi Reh, Seoul 06567 (KR); KIM, Yeung Hyen, Hwaseong-si, Gyeonggi-do 18424 (KR); CHO, Seung Kee, Suwon-si, Gyeonggi-do 16225 (KR)
(74) Representative: Brevalex
(86) International application number: PCT/KR2019/018596
(87) International publication number: WO 2020/139014

(57) **Abstract**

Provided are a VIP gene-expressing microorganism, a composition comprising the microorganism for prevention or treatment of a disease causative of gastrointestinal damage, a kit, and a method for prevention or treatment of a disease causative of gastrointestinal damage.

## Description

### Technical Field

The present disclosure relates to a microorganism expressing VIP gene, a composition or a kit for preventing or treating a disorder causing gastrointestinal tract damage comprising the microorganism, and a method for preventing or treating a disorder causing gastrointestinal damage using the same.

### Background Art

Vasoactive intestinal peptide (VIP) is a peptide hormone that is vasoactive in the intestine. VIP is a peptide of 28 amino acid residues that belongs to a glucagon/secretin superfamily, the ligand of class II G protein-coupled receptors.

In humans, chromosome 6 q25 region on human genome codes for secretin family member having the length of 170 amino acids. The member composed of 170 amino acids is post-translationally cleaved to form vasoactive intestinal peptide (VIP). An active form of VIP polypeptide functions to lower blood pressure, increase vasodilation, relax smooth muscle in the respiratory system and the gastrointestinal tract tissue, reduce immune responses by not only the reduction of Thl response but also the promotion of Th2 response, modulate innate and adaptive immune responses, or promote electrolyte secretion in the gut. In addition, VIP is reported to be active in the communication with lymphocytes in central nerve system as a neurotransmitter. The bioactivity of VIP is transduced via three known receptors: VIP₁R, VIP₂R, and PAC₁R. These receptors are known to cause the production of intracellular calcium as well as the accumulation of cAMP. Their affinity to secretin depends on their subtypes and amino acid sequences of ligands like VIP. Human natural VIP has a short half-life of about 2 minutes in the blood stream.

United States Patent No. 9,561,262 discloses a method for treating hypertension in a patient, comprising administering VIP and an anti-hypertension agent to the patient.

United States Publication No. 2016-0045557 A1 discloses *Lactobacillus* and *Bifidobacterium* strains increasing the level of VIP as probiotics for improving enteric nerve system. However, these strains were screened in the co-culture system comprising epithelial cells and enteric neuronal cells, so they are not genetically engineered strains.

Chinese Publication No. 108753670 A discloses a multifunctional complex micro-ecological formulation, nano-selenium-recombinantly expressed *VIP-Lactococcus lactis,* and a preparation method thereof. To express VIP which is an antibacterial peptide, *Lactococcus lactis,* and NICE® expression system using nisin inducible promoter and USP45 signal sequence were employed. It also discloses its use as animal feed additive.

Despite the prior arts, there is still a need for a microorganism expressing VIP gene and its use for the treatment of inflammatory intestinal disorders.

### Disclosure of Invention

### Technical Problem

An aspect provides a recombinant microorganism genetically modified to increase the expression of VIP encoding gene.

Another aspect provides a composition for preventing or treating a disorder causing gastrointestinal tract damage in human, comprising the microorganism.

Still another aspect provides a kit for use in preventing or treating a disorder causing gastrointestinal tract damage, comprising the microorganism and a TNF-alpha blocker.

Further still another aspect provides a method for preventing or treating a disorder causing gastrointestinal tract damage.

### Solution to Problem

As used herein, the term, "VIP" or "VIP protein" or "VIP polypeptide," indicates a biologically active polypeptide having one or more biological activities as described herein. VIP as described herein includes any variant mimicking functions of natural VIP. The variant includes ones having an increased half-life and equivalent or higher biological activities as compared with natural VIP.

VIP as used herein includes, but is not limited to, human VIP, recombinant human VIP, murine VIP and/or recombinant murine VIP. The VIP may have the sequence homology of 50% or more, 60% or more, 70% or more, 85% or more, 90% or more, 95% or more, 98% or more, 99% or more, or 100% with the amino acid sequence of SEQ ID No. 1. The VIP may have any of amino acid sequences of SEQ ID Nos. 1, 2, 3, 4, 5, 6, 7, 8, and 9, respectively. VIP of SEQ ID No. 2 is human natural VIP. VIPs of SEQ ID Nos. 1, 3, 4, 5, 6, 7, 8, and 9 are variants of human natural VIP, i.e., VIP1, VIP2, VIP3, VIP4, VIP5, VIP6, VIP7, and VIP8, respectively.

VIP polypeptide as used herein could be isolated from various sources such as human tissues or other sources or prepared by recombination or synthetic methods. The term, "VIP polypeptide," includes any variant of VIP polypeptide. VIP as used herein includes chimeric forms such as VIP fused with another heterologous polypeptide or amino acid sequence.

An aspect provides a recombinant microorganism genetically modified to increase the expression of VIP encoding gene.

The microorganism may comprise a genetic modification to increase the expression of VIP. The genetic modification may be one to increase the copy number of VIP encoding gene. The increased copy number could be obtained by introduction of a heterologous gene to the microorganism. The introduction could be accomplished by transformation, transduction, transfection or electroporation. The introduced heterologous gene may or may not be integrated to the genome of host cells.

The microorganism may comprise a heterologous gene encoding VIP. The gene may be operably linked to a regulatory sequence regulating its expression. The regulatory sequence may be any nucleotide sequence encoding a promoter, an operator, a terminator, or a signal peptide. The promoter may a constitutive promoter. The promoter may have a transcription initiation rate equivalent to or higher than that of an art-known promoter, for example, promoter P11, in lactic acid bacteria. The promoter may be an inducible promoter. The inducible promoter may be, for example, a nisin inducible promoter. The promoter may be PR4 derived from *Lactobacillus paracasei.* The PR4 promoter may have the nucleotide sequence of SEQ ID No. 10. The PR4 promoter is set forth in International Publication No. WO 2019/132231 A1 filed on November 8, 2018, the content of which is incorporated hereinto in its entirety by reference. In case a nucleotide sequence encoding the signal peptide is in-frame linked to the heterologous protein gene, the signal peptide may have an increased capability to secrete the heterologous protein extracellularly compared with an art-known signal peptide, for example, signal peptide USP45. The signal peptide may be SP4 derived from *Lactobacillus paracasei.* SP4 may have the amino acid sequence of SEQ ID No. 11. SP4 is set forth in International Publication No. WO 2019/132231 A1 filed on November 8, 2018, the content of which is incorporated hereinto in its entirety by reference. The microorganism could secrete VIP extracellularly.

The microorganism may be a bacterium. The bacterium may be a gram-positive or -negative bacterium. The gram-positive bacterium may a lactic acid bacterium. The lactic acid bacterium may belong to the genus of *Lactobacillus, Lactococcus, Bifidobacterium, Streptococcus, Leuconostoc, Weissella, Pediococcus,* or *Enterococcus.* The lactic acid bacterium may be *Lactobacillus paracasei, Lactobacillus brevis, Lactobacillus plantarum* or *Lactococcus lactis.* The lactic acid bacterium may be *Lactobacillus plantarum* LMT1-9 (KCTC 13421BP), *Lactobacillus paracasei* LMT1-21 (KCTC 13422BP), or *Lactobacillus brevis* LMT1-46 (KCTC 13423BP).

Another aspect provides a composition for preventing or treating a disorder causing gastrointestinal tract damage, comprising the microorganism.

The disorder may cause gastrointestinal tract inflammation. The disorder may be one or more selected from the group consisting of inflammatory bowel disease (IBD), and colitis. The inflammatory bowel disease may be ulcerative colitis, or Crohn's disease.

The composition may further comprise one or more pharmaceutically or sitologically acceptable carriers, excipients, or diluents. The pharmaceutically or sitologically acceptable carrier includes any standard pharmaceutically or sitologically acceptable carrier such as phosphate buffered saline solution, 5% dextrose aqueous solution and emulsion (for example, oil/water or water/oil emulsion). Non-limiting examples of the excipients may include auxiliaries, binders, fillers, diluents, disintegrating agents, emulsifying agents, wetting agents, gliadants, lubricants, sweetening agents, flavors, and coloring agents. It depends on an intended administration route of an active ingredient which pharmaceutically or sitologically acceptable carrier is preferable. Typical administration route may include enteral (for example, oral) administration. The composition may be a unit dosage form. The composition may be an orally administrable formulation. The composition may be a food or a pharmaceutical composition. The composition may comprise a dried product of the microorganism. The composition may comprise a culture medium of the microorganism.

As used herein, the term, "pharmaceutically or sitologically acceptable," means that any adverse reaction does not substantially occur upon administration to an individual. The adverse reaction may be toxicity, allergy, or an immune response.

Another aspect provides a kit for preventing or treating a disorder causing gastrointestinal tract damage comprising the recombinant microorganism and any other therapeutic agent for a disorder causing gastrointestinal tract damage. For the recombinant microorganism, the same is applied as above. The recombinant microorganism may be in the form of the above-described composition. The kit may include a written instruction to use the recombinant microorganism and any other therapeutic agent for a disorder causing gastrointestinal tract damage in preventing or treating a disorder causing gastrointestinal tract damage.

Examples of any other therapeutic agent for gastrointestinal tract damage include any agents for treating a disorder causing gastrointestinal tract damage, for example, inflammatory bowel disease. The agent for treating a disorder causing gastrointestinal tract damage may include one or more of the following agents:
(i) Steroid anti-inflammatory agent
   Dexamethasone, hexestrol, methimazole, betamethasone, triamcinolone, triamcinolone acetonide, fluocinonide, fluocinolone acetonide, predonisolone, methylpredonisolone, cortisone acetate, hydrocortisone, fluorometholone, beclomethasone dipropionate, estriol, paramethasone acetate, fludrocortisone acetate, clobetasol propionate, diflorasone acetate, dexamethasone propionate, difluprednate, betamethasone dipropionate, budesonide, diflucortolone valerate, amcinonide, halcinonide, mometasone furoate, hydrocortisone butyrate propionate, flumetasone pivalate, clobetasone butyrate, dexametasone acetate or the like;
(ii) 5-aminosalicylic acid
   Sulfasalazine, mesalazine, olsalazine, balsalazide or the like;
(iii) Immunomodulator or immunosuppressant
   Methotrexate, cyclophosphamide, MX-68, atiprimod dihydrochloride, BMS-188667, CKD-461, rimexolone, cyclosporine, tacrolimus, gusperimus, azathiopurine, antilymphocyte serum, freeze-dried sulfonated normal immunoglobulin, erythropoietin, colony stimulating factor, interleukin, interferon or the like;
(iv) JAK inhibitor
   Tofacitinib, ruxolitinib or the like;
(v) TNF inhibitor
   Recombinant TNF-alpha-receptor IgG-Fc fusion protein (etanercept), infliximab, adalimumab, certolizumab pegol, golimumab, PASSTNF-α, soluble TNF-α receptor, TNF-α binding protein, anti-TNF-a antibody, CDP571 or the like;
(vi) Integrin inhibitor
   Natalizumab, vedolizumab, AJM300, TRK-170, E-6007 or the like;
(vii) Interleukin-12/23 inhibitor
   Ustekinumab, briakinumab (anti-interleukin-12/23 antibody) or the like;
(viii) Non-steroid anti-inflammatory drug (NSAID)
   (a) Classical NSAID
      Alcofenac, aceclofenac, sulindac, tolmetin, etodolac, fenoprofen, thiaprofenic acid, meclofenamic acid, meloxicam, tenoxicam, lornoxicam, nabumeton, acetaminophen, phenacetin, ethenzamide, sulpyrine, antipyrine, migrenin, aspirin, mefenamic acid, flufenamic acid, diclofenac sodium, ketophenylbutazone, loxoprofen sodium, phenylbutazone, indomethacin, ibuprofen, ketoprofen, naproxen, oxaprozin, flurbiprofen, fenbufen, pranoprofen, floctafenine, piroxicam, tenoxicam, epirizole, tiaramide hydrochloride, zaltoprofen, gabexate mesylate, camostat mesylate, ulinastatin, colchicine, probenecid, sulfinpyrazone, bucolome, benzbromarone, allopurinol, sodium aurothiomalate, hyaluronate sodium, sodium salicylate, salicylic acid, atropine, scopolamine, levorphanol, oxymorphone or a salt thereof or the like.
   (b) Cyclooxygenase inhibitor (COX-1 selective inhibitor, COX-2 selective inhibitor, etc.) Salicylic acid derivatives (e.g., celecoxib, aspirin), etoricoxib, valdecoxib, diclofenac sodium, indomethacin, loxoprofen or the like
   (c) Nitric oxide-releasing NSAIDs.

In addition, chemokine inhibitors, Cluster of Differentiation (CD) inhibitors, Interleukin inhibitors, RIP kinase inhibitors, smad7 inhibitors, MadCAM inhibitors or the like may be included.

Another aspect provides a method for preventing or treating a disorder causing gastrointestinal tract damage, comprising administering a therapeutically or prophylactically effective amount of the microorganism to an individual.

The method may further comprise administering a therapeutically or prophylactically effective amount of a TNF-alpha blocker to an individual. The TNF-alpha blocker may be administered simultaneously with, prior to, or following the administration of the microorganism. The TNF-alpha blocker may be administered at the same route as or different route from the microorganism. For example, the TNF-alpha blocker may be administered orally or parenterally. The parenteral administration may be injection such as intravenous, intraperitoneal or subcutaneous injection. For example, the TNF-alpha blocker may be administered intraperitoneally, while the microorganism may be administered orally. The TNF-alpha blocker can be intraperitoneally administered after the microorganism is orally administered. The TNF-alpha blocker can be administered at a single dose of 5 mg to 100 mg, 5 mg to 50 mg, 5 mg to 40 mg, 10 mg to 50 mg, or 20 mg to 50 mg.

In the above-described method, the disorder may be one that causes gastrointestinal tract inflammation. The disorder may be one or more selected from the group consisting of inflammatory bowel disease, autoimmune disease, radiation induced gastrointestinal tract damage or graft versus host disease (GVHD), inflammatory bowel disease (IBD), and colitis such as chronic colitis. The inflammatory bowel disease may be ulcerative colitis, or Crohn's disease.

As used herein, the term, "administering" or "administration," indicates a behavior to give the microorganism, the composition comprising the microorganism or a therapeutic treatment to a physiological system (e.g. an individual, or in vivo, in vitro or ex vivo cells, tissues or organs). Accordingly, in the method, the microorganism can be in the form of above-described composition. Examples of acceptable routes to human body may include oral, or mucosal administration, e.g. to enteral mucous, oral mucosa or buccal. The administration may be performed in combination with an additional therapeutic agent. The combinational administration includes administration in any order among simultaneous and sequential ones.

As used herein, the term, "treatment," indicates prophylactic treatment or therapeutic treatment. In a specific embodiment, "treatment" indicates administration of the microorganism or the composition to an individual for therapeutic or prophylactic purpose.

"Therapeutic" treatment is an administration to an individual showing pathological signs or symptoms for reducing or removing the signs or symptoms. The signs or symptoms may be biochemical, cellular, histological, functional or physical, subjective or object ones.

"Prophylactic" treatment is an administration to an individual showing no or only early pathological signs for reducing a risk of developing pathology. The microorganism or the composition as described herein can be provided for a prophylactic treatment to reduce the likelihood of pathology development or, if pathology has been developed, minimizing its severity.

As used herein, "therapeutically effective amount" indicates an amount sufficient to accomplish the described purpose. The effective amount can be determined experimentally. The effective amount can be determined depending on the extracellular secretion rate of VIP by the microorganism. For example, the effective amount may be the number of microorganisms which is capable of secreting 0.01 to 300 mg, or 0.5 to 100 mg of VIP daily for a human of 60 kg body weight. For example, it may be 1X10⁷ to 1X10¹¹ cfu, 1X10⁷ to 1X10¹⁰ cfu, 1X10⁸ to 1X10¹¹ cfu, 1X10⁸ to 1X10¹⁰ cfu, 2.5X10⁸ to 7.5X10⁹ cfu, 5.0X10⁸ to 5.0X10⁹ cfu, 7.5X10⁸ to 2.5X10⁹ cfu, or about 1X10⁹ cfu each administration to each individual.

In the above-described method, the individual may be a mammal. The mammal may be human, horse, pig, cow, dog, cat, monkey, chimpanzee, sheep, or goat. The individual may be human. The individual may be a non-human mammal.

### Advantageous Effect

The recombinant microorganism according to one aspect can produce and secrete VIP.

The composition or the kit according to another aspect can be used for preventing or treating a disorder causing gastrointestinal tract damage.

The method according to still another aspect can efficiently prevent or treat a disorder causing gastrointestinal damage.

### Brief Description of Drawings

Fig. 1 is a construction map of pMT447 vector.
Fig. 2 shows the results of western blotting of culture supernatant of *L. plantarum* LMT1-9, *L. paracasei* LMT1-21, and *L. brevis* LMT1-46 strains transformed with pMT447 vector.
Fig. 3 shows the therapeutic efficacy of VIP expressed from *L. plantarum* LMT1-9 transformed strain as DAI score in DSS induced mouse intestinal inflammation model.
Fig. 4 shows the therapeutic efficacy of VIP expressed from *L. paracasei* LMT1-21 transformed strain as DAI score in DSS induced mouse intestinal inflammation model.
Fig. 5 shows the therapeutic efficacy of VIP expressed from *L. brevis* LMT1-46 transformed strain as DAI score in DSS induced mouse intestinal inflammation model.
Fig. 6 shows the therapeutic efficacy of VIP expressed from transformed strain as DAI AUC score in DSS induced mouse intestinal inflammation model.
Fig. 7 shows the therapeutic efficacy of VIP expressed from transformed strain histopathologically in DSS induced mouse intestinal inflammation model.
Fig. 8 shows the therapeutic efficacy of combination of VIP expressed from transformed LAB strains and etanercept (Enbrel, Pfizer Korea) as DAI score in DSS induced mouse intestinal inflammation model.
Fig. 9 shows the therapeutic efficacy of combination of VIP expressed from transformed LAB strains and Enbrel as DAI AUC score in DSS induced mouse intestinal inflammation model.
Fig. 10 shows the therapeutic efficacy of combination of VIP expressed from transformed strain and Enbrel histopathologically in DSS induced mouse intestinal inflammation model.

### Best mode

Hereunder, detailed descriptions are provided with reference to examples. However, the examples are provided only for illustrative purpose, but not intended to limit the scope of the present disclosure in any manner.

### Example 1: Lactic acid bacteria expressing VIP gene, and their therapeutic efficacy for IBD

In the present example, VIP gene was introduced to lactic acid bacterial cells, and it was tested if the gene was expressed and extracellularly secreted. Furthermore, the lactic acid bacterial cells transformed with VIP gene were orally administered to an individual and tested for improvement in symptoms to show their efficacy for treating IBD. As VIP, a human VIP variant consisting of the amino acid sequence of SEQ ID No. 1 was used. The VIP variant has 4 amino acid substitutions in human natural VIP consisting of the amino acid sequence of SEQ ID No. 2, thereby to have an increased half-life in the human bloodstream.

### 1. Construction of VIP gene-containing vector

DNA synthesized by Macrogen, Inc. (South Korea) was used as VIP gene consisting of the nucleotide sequence of SEQ ID No. 14. The synthesized VIP gene fragment and Lactic Acid Bacteria (LAB)-*E*. *coli* shuttle vector pMT54-PR4-SP4 (SEQ ID No. 13) were cleaved with restrictive endonucleases Sall and Xhol, respectively. The cleaved gene fragment and vector in the reaction mixture were purified using a gel purification kit (GeneAII Biotechnology, Co., Ltd.), and then, were incubated in the presence of an alkaline phosphatase to obtain dephosphorylated cleaved VIP gene fragment and cleaved pMT54-PR4-SP4 vector.

The vector DNA of 1 µl, the VIP gene DNA of 3 µl, 0.5 µl of T4 DNA ligase (Takara), 1 µl of a buffer solution, and 5.5 µl of distilled water were mixed together in a test tube to make a total volume to 10 µl, and then, the mixture was incubated at 16°C for 12 hours to link the VIP gene with the vector fragment to obtain the VIP gene-containing vector. *E. coli* competent cells (TOP10 Competent Cells) were transformed with the vector according to the method of Sambrook et al. (Sambrook et al., Molecular Cloning: A Laboratory Manual, 2nd edn, 1989).

The transformed *E. coli* was spread onto an LB (Luria-Bertani)-agar plate containing 10 µl/ml of chloramphenicol, and then, incubated. As a result, formed colonies were selected, cultivated, and the vector was isolated from the culture. The vector was designated as pMT447. The pMT447 vector comprises the VIP gene operably linked to PR4 promoter and SP4 signal sequence. Therefore, the cells transformed with pMT447 vector expresses and secretes the VIP gene extracellularly in a high efficiency.

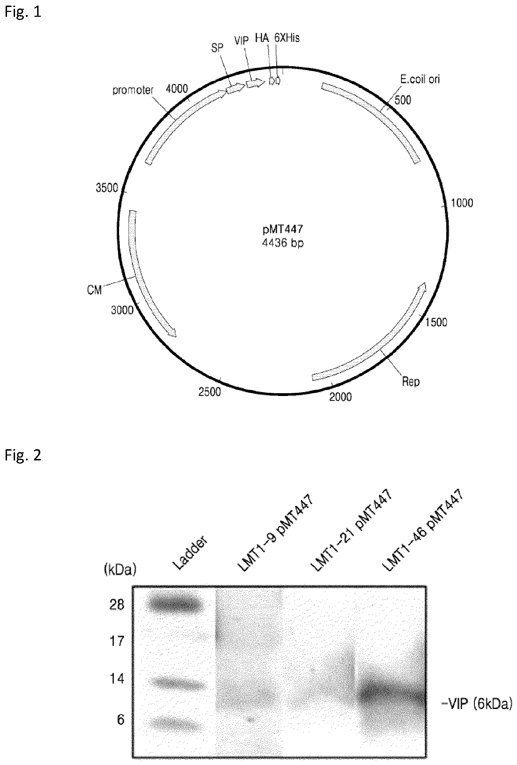

Fig. 1 shows the construction of pMT447 vector. In Fig. 1, the promoter is the sequence (SEQ ID No. 10) of PR4 promoter, SP is the nucleotide sequence (SEQ ID No. 11) of SP4 signal peptide, and VIP is the sequence (SEQ ID No. 14) of gene encoding human VIP variant of SEQ ID No. 1. HA and 6X His is the nucleotide sequence of human influenza hemagglutinin tag and 6 histidine tag for detection, isolation, and purification of protein. *E. coli* ori, Rep, and CM are the replication origin of *E. coli,* the replication origin of lactic acid bacteria, and gene for chloramphenicol resistance marker, respectively.

### 2. Introduction of recombinant vector to lactic acid bacterial cells

The recombinant vector pPM447 constructed in the above 1 were introduced to *Lactobacillus plantarum* LMT1-9 (KCTC 13421BP), *Lactobacillus paracasei* LMT1-21 (KCTC 13422BP), and *Lactobacillus brevis* LMT1-46 (KCTC 13423BP), respectively, and then, were tested for the secretion of VIP protein.

Each strain was cultivated to reach OD₆₀₀ of 0.5 in a flask containing 50 mL of MRS medium (Difco Co., USA), and then, centrifuged at 4°C, 7,000 rpm for 10 minutes. Cell pellets were washed with 25 mL of cold EPS (ice-cold electroporation solution) (containing 1 mM K₂HPO₄, 1mM KH₂PO₄, pH 7.4,1 mM MgCl₂, and 0.5 M sucrose) twice. MRS is also called as De Man, Rogosa and Sharpe agar medium.

After washing, the cells were re-suspended in 1 ml of cold EPS to prepare competent cells for electroporation and kept in a deep freezer of -80°C. The competent cells of 40 µl and the respective vector DNA (1 µg/µl) of 1 µl were added to a cuvette, and allowed to stand in ice for 5 minutes. Electric pulse was imposed on the cells under the condition of 25 µF, 8 kV/cm, 400 ohms, and then, the cells were immediately added to 1 ml of MRS liquid medium and cultivated at 37°C for one hour. Then, the cultivated cells were spread onto MRS medium (also known as 'MRS-CM') containing 10 µg/ml of chloramphenicol and cultivated at 37°C for 48 hours.

The obtained pMT447 vector-introduced strains were statically cultivated in MRS liquid medium at 37°C for 16 hours, respectively. The culture was inoculated to 3% (v/v) in MRS liquid medium, and then, statically cultivated for 8 hours at the same temperature. The culture of 1 ml was centrifuged at 7,000 rpm for 5 minutes to take its supernatant. To the supernatant of 1 ml was added trichloroacetic acid of 100 µl to obtain a mixture and the mixture was allowed to stand at 4°C for one hour to concentrate the culture. The culture was centrifuged at 13,000 rpm for 4°C, and the pellets were washed with 1 ml of cold acetone, dried at room temperature for 10 minutes, and eluted with Tris-HCI buffer (pH 8.8) of 100 µl.

To the elute were added 4X loading buffer (Thermo) and 10X reducing agent (Thermo), and electrophoresis was performed on SDS-PAGE gel. The gel was transferred onto nitrocellulose membrane with Trans blot semi-dry cell (bio-rad) to perform western blotting. Specifically, the membrane was blocked with TBST buffer containing 1% skim milk for one hour, reacted with anti-HA antibody (Santa cruz) at room temperature for 2 hours, and then, washed with TBST for 5 minutes three times and detected with ECL. In pMT447 vector, VIP gene was operably liked with hemagglutinin (HA) gene at its 3' terminal, so was expressed in HA tagged form.

Fig. 2 shows the results of western blotting of culture supernatants of pMT447 vector-transformed *L. plantarum* LMT1-9, *L. paracasei* LMT1-21, and *L. brevis* LMT1-46 strains. In Fig 2, lanes 2, 3, and 4 represent pMT447 vector-transformed *L. plantarum* LMT1-9, *L. paracasei* LMT1-21, and *L. brevis* LMT1-46, respectively, and lane 1 represents a standard protein ladder.

As shown in Fig. 2, the transformed strains efficiently secreted hVIP variants.

### 3. Efficacy of VIP-secreting lactic acid bacteria for IBD in DSS induced model

The transformed *L. plantarum* LMT1-9, *L. paracasei* LMT1-21, and *L. brevis* LMT1-46 as described in 2 were orally administered to mouse colitis model induced with dextran sulfate sodium salt (DSS), and the survival rate and disease activity index (DAI) score of mice were measured to evaluate the therapeutic efficacy of the strains on intestinal inflammation.

### (1) Administration of recombinant microorganisms alone

Specifically, the transformed strains were statically cultivated primarily in a flask containing MRS medium of 10 ml containing 10 µg/ml of chloramphenicol at 37°C for 24 hours. On the next day, the cultivated strains were inoculated on 300 to 600 ml of MRS CM medium to reach OD₆₀₀ of 0.01, and secondarily cultivated under the same condition as the primary cultivation. After 16 to 18 hours, when OD₆₀₀ of the culture reached 2 to 3, the culture was centrifuged at 7,000 rpm for 10 minutes to discard the supernatant, and cells were suspended in 1X PBS so as to be administered at 1X10⁹ cfu as a single dose per mouse. This cell-containing PBS solution was administered orally (1X10⁹ cfu as a single dose per mouse) with drinking water once a day for 16 days.

First, male Balb/c mice with the age of 8 to 10 weeks (20 to 25 g, 10 mice per group) (ORIENTBIO) were used as experimental animals. The day when strains were administered initially was set Day -7, and DSS diluted to 2% (v/v) in distilled water was supplied as drinking water on Day 0 to Day 6 to all groups but PBS group. Mice took drinking water voluntarily, but at an amount of 6 ml or less a day generally. The mice were sacrificed on Day 9.

After DSS was administered, survival rate was measured every other day and body weight, hair-raising, movement, and diarrhea were checked to obtain DAI score.

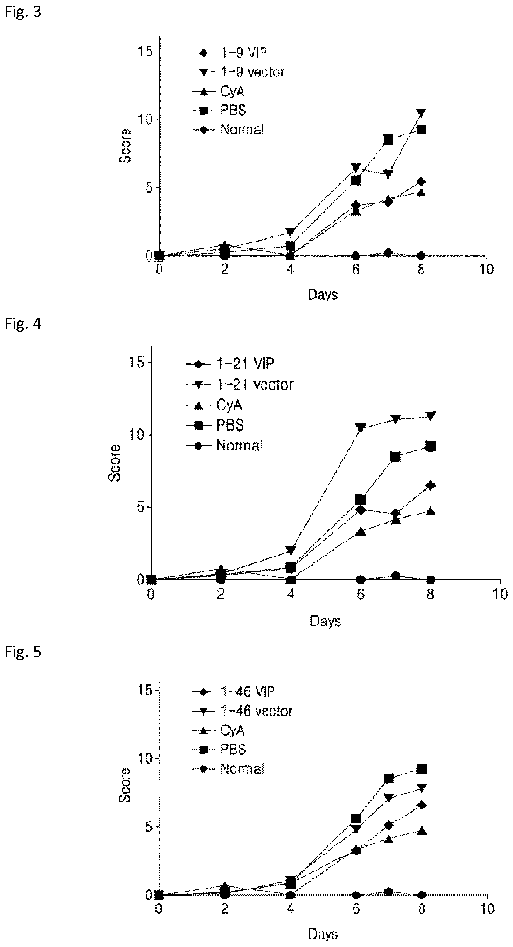

Disease activity index (DAI) scoring was performed with reference to Ameho, Gut 1997; 41:487^{∼}493 and Wallace, Gastroenterology 1989; 96: 29^{∼}36. The results were shown in Fig. 3 to Fig. 6.

Fig. 3 shows the therapeutic efficacy of VIP expressed from the transformed *L*. *plantarum* LMT1-9 strain in DSS-induced mouse intestinal inflammation model as DIA score. In Fig. 3, 1-9 VIP represents pMT447-transformed *L. paracasei* LMT1-9, and 1-9 vector represents control vector (parental vector having no VIP gene)-transformed *L. plantarum* LMT1-9.

Fig. 4 shows the therapeutic efficacy of VIP expressed from the transformed *L*. *plantarum* LMT1-21 strain in DSS-induced mouse intestinal inflammation model as DIA score. In Fig. 4, 1-21 VIP represents pMT447-transformed *L. plantarum* LMT1-21, and 1-21 vector represents control vector (parental vector having no VIP gene)-transformed *L. plantarum* LMT1-21.

Fig. 5 shows the therapeutic efficacy of VIP expressed from the transformed *L. brevis* LMT1-46 in DSS-induced mouse intestinal inflammation model as DIA score. In Fig. 5, 1-46 VIP represents pMT447-transformed *L. brevis* LMT1-46, and 1-46 vector represents control vector (parental vector having no VIP gene)-transformed *L. brevis* LMT1-46.

In Fig. 3 to Fig. 5, CyA is the abbreviation of cyclosporine A, which was used as a positive control of the experiments. PBS represents a control group treated with DSS, not with the bacteria. Normal represents a group treated neither DSS nor the bacteria.

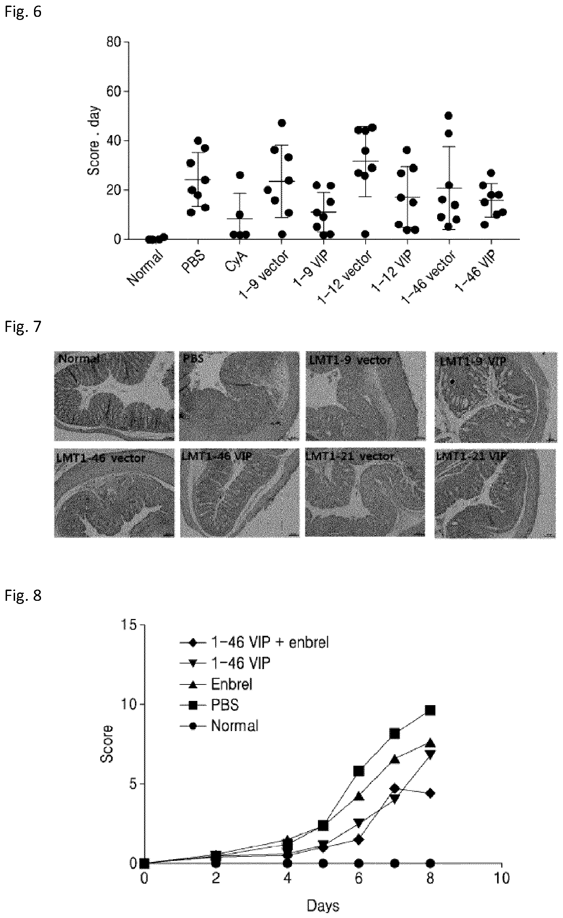

Fig. 6 shows the therapeutic efficacy of VIP expressed from the transformed strains as DAI AUC score in DSS-induced mouse intestinal inflammation model. Each strain and chemical are as described above and DAI AUC represents area under curve of DAI score until Day 8.

As shown in Fig. 3 to 6, the strains transformed to express VIP showed the therapeutic efficacy in DSS-induced intestinal inflammation model.

Fig. 7 shows the therapeutic efficacy of VIP expressed from the transformed strains histopathologically in DSS-induced mouse intestinal inflammation model. In Fig. 7, Normal, PBS, LMT1-9 vector, LMT1-9 VIP, LMT1-46 vector, LMT1-46 VIP, LMT1-21 vector, and LMT1-21 VIP are as described for Figs. 1 to 6.

As shown in Fig. 7, mice administered with VIP-expressing *Lactobacillus* strains showed the therapeutic efficacy compared with the control group in DSS-induced intestinal inflammation mouse model, as demonstrated by histopathological examination on the colon with a microscope.

Specifically, as shown in Fig. 7, histopathological examination was performed with a microscope on the colon in DSS-induced intestinal inflammation mouse model. As a result, while monocyte infiltration, mucosal damage, etc. were the severest in DSS group and no significant effect was observed in LMT 1-9 vector, LMT 1-21 vector, and LMT 1-46 vector administration groups, histological damage and inflammatory infiltration were reduced in LMT 1-9 VIP, LMT 1-21 VIP, and LMT1-46 VIP administration groups. This suggests that the VIP-expressing *Lactobacillus* strains have inflammation ameliorating and protective effects in DSS-induced inflammatory bowel disease mouse model.

### (2) Effects of the combination of the recombinant microorganism and a TNF-alpha blocker

Fig. 8 shows the therapeutic efficacy of the combination of VIP expressed from transformed LAB strains and etanercept (Enbrel, Pfizer Korea) as DAI score. In Fig. 8, Normal represents non-treatment group. PBS represents only DSS-treatment group. Enbrel represent DSS and Enbrel-treatment group. 1-46 VIP represents a group administered with *L. brevis* LMT1-46 transformed with pMT447 in DSS-induced mice. 1-46 VIP + Enbrel represents a group administered with *L. brevis* LMT1-46 transformed with pMT447 and Enbrel together in DSS-induced mice.

Fig 8 shows the results obtained by the following procedures. To evaluate the therapeutic efficacy of VIP expressed from transformed strains, the transformed stains were orally administered to DSS-induced mouse intestinal inflammatory model to measure the survival rate and DAI score. The transformed strains were primarily cultivated in 10 ml of MRS medium containing 10 µl/ml chloramphenicol for one day, and the cultivated strains were inoculated onto 300 to 600 ml of MRS CM medium to reach OD₆₀₀ of 0.01. After 16 to 18 hours, when OD₆₀₀ of the culture was reached 2^{∼}3, the strains were recovered considering the number of mice and administration times. The culture supernatant was discarded by centrifugation at 7,000 rpm for 10 minutes, and the recovered strains were suspended in 1X PBS to be administered at 1X10⁹ cfu as a single dose per mouse. The strain administration was performed once a day for 16 days in total.

The initial administration day was set Day -7 to start the therapeutic efficacy evaluation test. DSS was diluted to 2% (v/v) in distilled water, which was supplied to all groups but PBS group as drinking water over Day 0^{∼} Day 6. On Day 9, mice were euthanized. The survival rate was checked every other day since DSS treatment, and body weight, hair raising, animal's movement, diarrhea were checked to calculate DAI score. To evaluate the efficacy of the combination with Etanercept (TNF blocker; Enbrel), Enbrel was intraperitoneally administered to the group at a dosage of 10 mg/kg on Day 0, 2, 4, and 6.

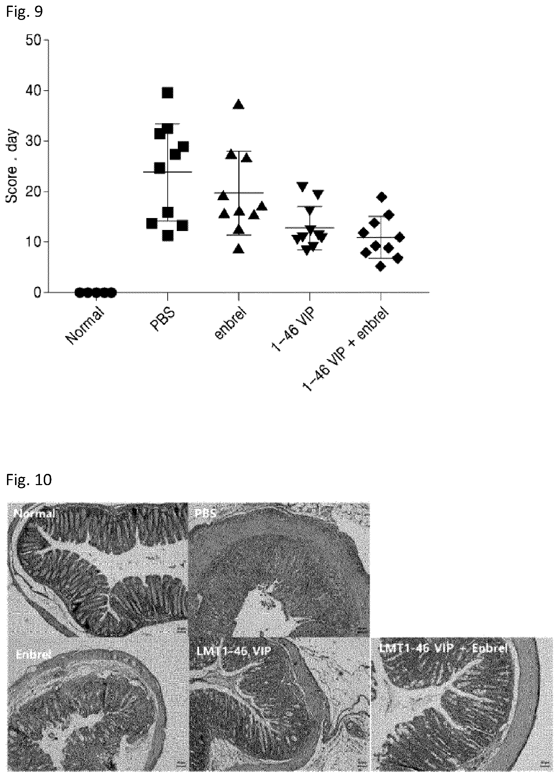

Fig. 9 shows the therapeutic efficacy of the combination of VIP expressed from transformed LAB strains and Enbrel as DAI AUC score in DSS-induced mouse intestinal inflammation model. Each strain and each chemical were as described above. DAI AUC represents the area under the curve of DAI score until Day 8.

As shown in Fig. 8 and Fig. 9, the group treated with combination of the VIP expressing strains and Enbrel showed reduced DAI score compared with the strain treatment group or Enbrel treatment group.

Enbrel used in this experiment is only an illustrative agent for treating inflammatory bowel disease, and other agents for treating inflammatory bowel disease could be used in combination with VIP expressed from transformed LAB strains.

### (3) Histopathology test

The animals were euthanized on the 9^{th} day after starting the experiment. Two parts with the distance of 2 cm in the distal area of the colon were extracted at the length of 0.5 cm, respectively, and were fixed in 10% formalin solution and paraffin embedded. The embedded tissue was cut with a microtome to pieces having the length of 5 µm to prepare slides and stained with H&E. The stained tissue was tested for severity with referring to the evaluation method of Erben et al., Int J Clin Exp Pathol 2014;7(8):4557-4576, which was performed three, or four or more random parts per sample.

The evaluation method may be briefly described as below. Intestinal epithelial cells damage and inflammatory cell infiltration was separately evaluated and two scores were summed to obtain scores ranging from 0 to 8.

Inflammatory cell infiltration was evaluated according to the following standard:
0 = normal,
1 = mild-infiltrate around crypt basis,
2 = moderate-infiltrate reaching to L. muscularis mucosae,
3 = marked -extensive infiltration reaching the muscularis mucosae and thickening of the mucosa,
4 = severe-infiltration of the L. submucosa.

Epithelial cell damage was evaluated according to the following standard:
0 = normal,
1 = focal erosions-loss of goblet cells,
2 = erosions- loss of goblet cells in large areas,
3 = erosion/ulceration-loss of crypts (focal),
4 = extended ulceration-granulation tissue, loss of crypts in large areas.

Fig. 10 shows the therapeutic efficacy of the combination of VIP expressed from transformed LAB strains and Enbrel histopathologically in DSS-induced mouse intestinal inflammation model. In Fig. 10, Normal, PBS, Enbrel, LMT1-46 VIP, and LMT1-46 VIP+Enbrel are as described for Fig. 9.

As shown in Fig. 10, the group administered with the combination of VIP-expressing strains and Enbrel showed improved therapeutic effect compared with the group administered with Enbrel only.

Specifically, as shown in Fig. 10, histopathology examination was performed with a microscope on the colon in DSS-induced intestinal inflammation model. As a result, DSS group showed the severest monocyte infiltration and mucosal damage, and Enbrel treatment group showed not so much improvement on such pathologies. In contrast, LMT1-46 VIP treatment group as well as LMT 1-46 VIP & Enbrel combination treatment group showed reduced histological damage and inflammatory infiltration lesions as in CyA treatment group used as a positive control. This supports that the VIP-expressing *Lactobacillus* strains have inflammation ameliorating and protective effects in DSS-induced inflammatory bowel disease mouse model.

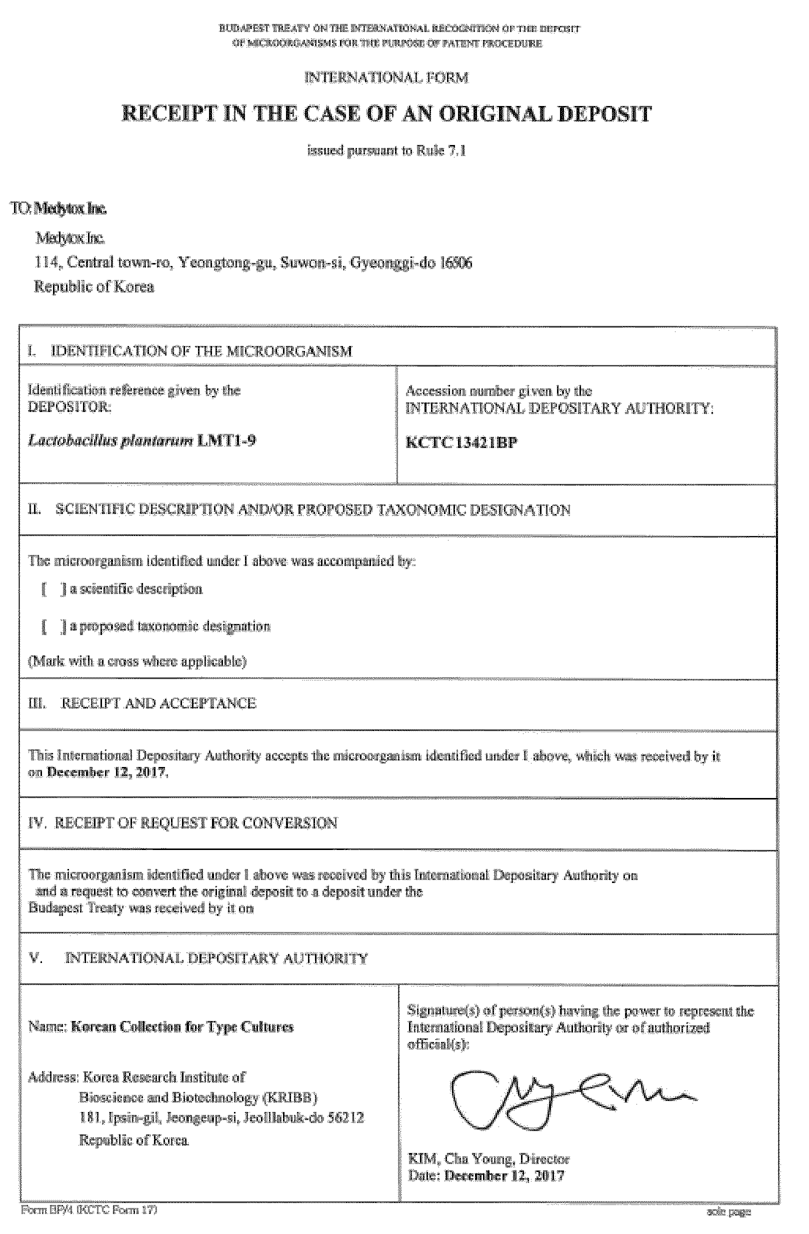
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: Medytox Inc.

Medytox Inc.
114, Central town-ro, Yeongtong-gu, Suwon-si, Gyeonggi-do 16506
Republic of Korea

I. IDENTIFICATION OF THE MICROORGANISM
Identification reference given by the
DEPOSITOR:

Lactobacillus plantarum LMT1-9 | Accession number given by the
INTERNATIONAL DEPOSITARY AUTHORITY:

KCTC 13421BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on December 12, 2017.
IV. RECEIPT OF REQUEST FOR CONVERSION
The microorganism identified under I above was received by this International Depositary Authority on
and a request to convert the original deposit to a deposit under the
Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):


KIM, Cha Young, Director
Date: December 12, 2017

Form BP/4 (KCTC Form 17)
sole page

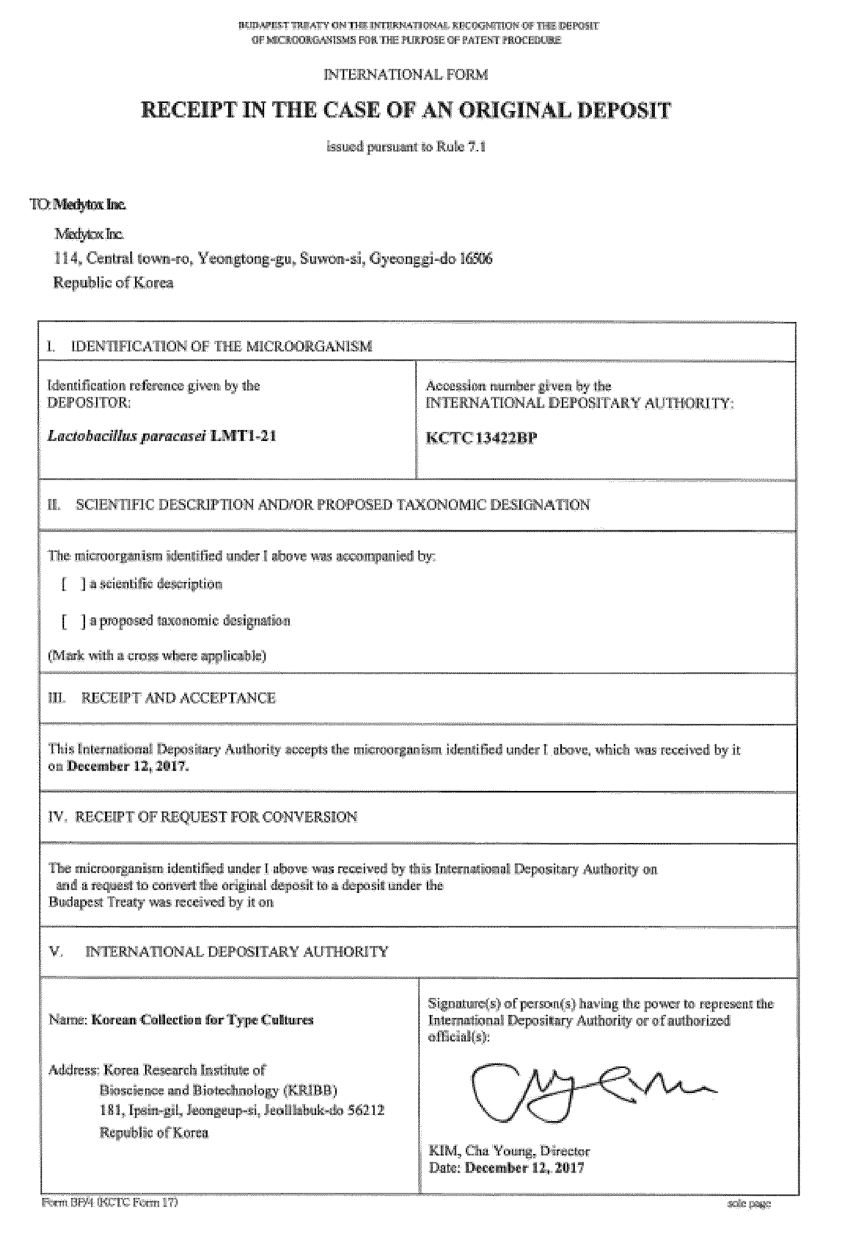
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: Medytox Inc.

Medytox Inc.
114, Central town-ro, Yeongtong-gu, Suwon-si, Gyeonggi-do 16506
Republic of Korea

I. IDENTIFICATION OF THE MICROORGANISM
Identification reference given by the
DEPOSITOR:

Lactobacillus paracasei LMT1-21 | Accession number given by the
INTERNATIONAL DEPOSITARY AUTHORITY:

KCTC 13422BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on December 12, 2017.
IV. RECEIPT OF REQUEST FOR CONVERSION
The microorganism identified under I above was received by this International Depositary Authority on
and a request to convert the original deposit to a deposit under the
Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):


KIM, Cha Young, Director
Date: December 12, 2017
Form BP/4 (KCTC Form 17) sole page

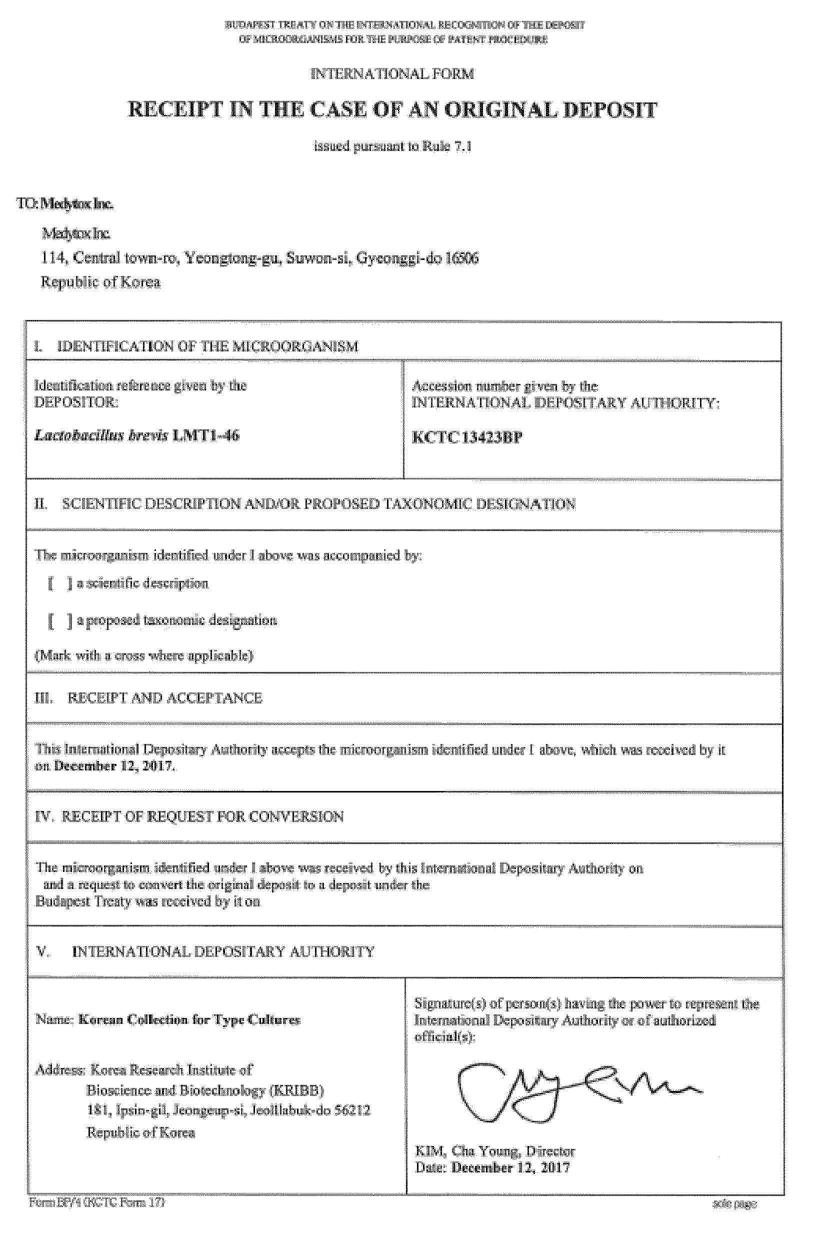
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: Medytox Inc.

Medytox Inc.
114, Central town-ro, Yeongtong-gu, Suwon-si, Gyeonggi-do 16506
Republic of Korea

I. IDENTIFICATION OF THE MICROORGANISM
Identification reference given by the
DEPOSITOR: | Accession number given by the
INTERNATIONAL DEPOSITARY AUTHORITY:
Lactobacillus brevis LMT1-46 | KCTC 13423BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on December 12, 2017.
IV. RECEIPT OF REQUEST FOR CONVERSION
The microorganism identified under I above was received by this International Depositary Authority on
and a request to convert the original deposit to a deposit under the
Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):
KIM, Cha Young, Director
Date: December 12, 2017
Form BP/4 (KCTC Form 17) sole page

## Claims

1. A recombinant microorganism, comprising a promoter, and a heterologous gene encoding vasoactive intestinal peptide (VIP) operably linked to the promoter, wherein the microorganism belongs to the genus of *Lactobacillus.*

2. The microorganism of Claim 1, which is *Lactobacillus paracasei, Lactobacillus brevis,* or *Lactobacillus plantarum.*

3. A recombinant microorganism, comprising a constitutive promoter, and a heterologous gene encoding vasoactive intestinal peptide (VIP) operably linked to the promoter, wherein the microorganism is a lactic acid bacterium.

4. The microorganism of Claim 3, wherein the constitutive promoter consists of the sequence as set forth in SEQ ID No. 10.

5. The microorganism of Claim 3, wherein the lactic acid bacterium belongs to the genus of *Lactobacillus,* or *Lactococcus.*

6. The microorganism of Claim 5, wherein the lactic acid bacterium is *Lactobacillus paracasei, Lactobacillus brevis, Lactobacillus plantarum,* or *Lactococcus lactis.*

7. The microorganism of any one of Claims 1 to 6, further comprising an operably linked signal sequence between the promoter and the heterologous gene.

8. The microorganism of Claim 7, wherein the signal sequence encodes a signal peptide consisting of the amino acid sequence as set forth in SEQ ID No. 11.

9. A composition for preventing or treating a disorder causing gastrointestinal tract damage in human, which comprises a recombinant microorganism comprising a promoter, and a heterologous gene encoding vasoactive intestinal peptide (VIP) operably linked to the promoter, wherein the microorganism is a lactic acid bacterium.

10. The composition of Claim 9, wherein the disorder causes gastrointestinal tract inflammation.

11. The composition of Claim 10, wherein the disorder is one or more selected from the group consisting of inflammatory bowel disease (IBD), and colitis.

12. The composition of Claim 11, wherein the inflammatory bowel disease is ulcerative colitis, or Crohn's disease.

13. The composition of Claim 9, which is an oral dosage form.

14. The composition of Claim 9, wherein the lactic acid bacterium belongs to the genus of *Lactobacillus,* or *Lactococcus.*

15. The composition of Claim 14, wherein the lactic acid bacterium is *Lactobacillus paracasei, Lactobacillus brevis, Lactobacillus plantarum,* or *Lactococcus lactis.*

16. The composition of any one of Claims 9 to 15, wherein the promoter is a constitutive promoter.

17. The composition of Claim 16, wherein the constitutive promoter consists of the nucleotide sequence of SEQ ID No. 10.

18. The composition of any one of Claims 9 to 15, wherein the recombinant microorganism further comprises an operably linked signal sequence between the promoter and the heterologous gene.

19. The composition of Claim 18, wherein the signal sequence encodes a signal peptide consisting of the amino acid sequence of SEQ ID No. 11.

20. The composition of any one of Claims 9 to 15, further comprising a therapeutic agent for treating a disorder causing gastrointestinal tract damage.

21. The composition of Claim 20, wherein the therapeutic agent is selected from the group consisting of steroid anti-inflammatory drug, 5-aminosalicylic acid, immunomodulatory agent, JAK inhibitor, TNF inhibitor, integrin inhibitor, interleukin inhibitor, chemokine inhibitor, cluster of differentiation inhibitor, interleukin, RIP kinase inhibitor, smad7 inhibitor, MadCAM inhibitor and non-steroid anti-inflammatory drug (NSAID).

22. The composition of Claim 21, wherein the non-steroid anti-inflammatory drug is classical NSAID, cyclooxygenase inhibitor, or nitric oxide-releasing NSAID.


SEQUENCE LISTING

<110>  MEDYTOX INC.

<120>  Microorganism expressing heterologous protein, and use thereof

<130>  PX060551OV

<150>  KR1020180173063
<151>  2018-12-28

<160>  14

<170>  PatentIn version 3.5

<210>  1
<211>  28
<212>  PRT
<213>  VIP variant 1

<400>  1

His Ser Asp Ala Val Phe Thr Asp Asn Tyr Thr Arg Leu Arg Arg Gln
1               5                   10                  15

Leu Ala Val Arg Arg Tyr Leu Asn Ser Ile Leu Asn
            20                  25

<210>  2
<211>  28
<212>  PRT
<213>  Homo sapiens

<400>  2

His Ser Asp Ala Val Phe Thr Asp Asn Tyr Thr Arg Leu Arg Lys Gln
1               5                   10                  15

Met Ala Val Lys Lys Tyr Leu Asn Ser Ile Leu Asn
            20                  25

<210>  3
<211>  30
<212>  PRT
<213>  Artificial

<220>
<223>  VIP Variant 2

<400>  3

His Ser Asp Ala Val Phe Thr Ala Asn Tyr Thr Arg Leu Arg Arg Gln
1               5                   10                  15

Leu Ala Val Arg Arg Tyr Leu Ala Ala Ile Leu Gly Arg Arg
            20                  25                  30

<210>  4
<211>  25
<212>  PRT
<213>  Artificial

<220>
<223>  VIP variant 3

<400>  4

Phe Thr Ala Asn Tyr Thr Arg Leu Arg Arg Gln Leu Ala Val Arg Arg
1               5                   10                  15

Tyr Leu Ala Ala Ile Leu Gly Arg Arg
            20                  25

<210>  5
<211>  28
<212>  PRT
<213>  Artificial

<220>
<223>  VIP variant 4

<400>  5

His Ser Asp Gly Ile Phe Thr Asp Ser Tyr Ser Arg Tyr Arg Lys Gln
1               5                   10                  15

Met Ala Val Lys Lys Tyr Leu Ala Ala Val Leu Asn
            20                  25

<210>  6
<211>  28
<212>  PRT
<213>  Artificial

<220>
<223>  VIP variant 5

<400>  6

His Ser Asp Ala Val Phe Thr Asp Asn Tyr Ala Arg Leu Arg Lys Gln
1               5                   10                  15

Met Ala Val Lys Lys Ala Leu Asn Ser Ile Leu Ala
            20                  25

<210>  7
<211>  31
<212>  PRT
<213>  Artificial

<220>
<223>  VIP variant 6

<400>  7

His Ser Asp Ala Val Phe Thr Asp Asn Tyr Thr Arg Leu Arg Lys Gln
1               5                   10                  15

Val Ala Ala Lys Lys Tyr Leu Gln Ser Ile Lys Asn Lys Arg Tyr
            20                  25                  30

<210>  8
<211>  31
<212>  PRT
<213>  Artificial

<220>
<223>  VIP variant 7

<400>  8

His Ser Asp Ala Val Phe Thr Asp Asn Tyr Thr Arg Leu Arg Arg Gln
1               5                   10                  15

Leu Ala Val Arg Arg Tyr Leu Asn Ser Ile Leu Asn Gly Arg Arg
            20                  25                  30

<210>  9
<211>  23
<212>  PRT
<213>  Artificial

<220>
<223>  VIP variant 8

<400>  9

His Ser Asp Ala Val Phe Thr Asp Asn Tyr Thr Arg Leu Arg Arg Gln
1               5                   10                  15

Leu Ala Val Arg Arg Tyr Leu
            20

<210>  10
<211>  500
<212>  DNA
<213>  Lactobacillus paracasei LMT1-21

<400>  10
tccacaatca aggtgcttgg ctttttcgat cgcgaggtca ccatgtacat cagtcgtgag      60

agcattgtgt tgacagtgat cggcatcgtg ttcggctatc tgctcggcaa tttgctgaca     120

gcctacattt tgtatcaagc cgaaactgag gccgtggttt ttccactcac gatcagcatt     180

gtcggctacc tcacggccac gttactcatg ttggccttca ccggcgtcgt cacctggctc     240

acgcatcgtc gactccaacg ggtggacatg gtcgaagccc tgaaatcaaa cgaataacct     300

acaattttgt caggcagcgt cgtcacggcg ctgctttttt catacaaaat tcatcaaaaa     360

ttgggattaa aaacgttcat gatcgcaatt ttgaagcgca aatgaagatt gagaccaact 420

cctaacagtc ctgtaacgct gacgtaacat tgacacagta aagtagcctt tagttaatca 480

aattaagggt gaggtcaaaa 500

<210> 11
<211> 31
<212> PRT
<213> Lactobacillus paracasei LMT1-21

<400> 11

Met Lys Phe Asn Lys Val Met Ile Thr Leu Val Ala Ala Val Thr Leu
1               5                   10                  15

Ala Gly Ser Ala Ser Ala Val Thr Pro Val Phe Ala Asp Thr Ser
            20                  25                  30

<210> 12
<211> 93
<212> DNA
<213> Lactobacillus paracasei LMT1-21

<400> 12
atgaaattca ataaagtcat gatcacgttg gttgctgcag ttaccttagc aggttctgct 60

agcgccgtaa caccagtttt cgctgataca agc 93

<210> 13
<211> 4259
<212> DNA
<213> Artificial

<220>
<223> pMT54-PR4-SP4 vector

<400> 13
ggtacctccg cttcctcgct cactgactcg ctgcgctcgg tcgttcggct gcggcgagcg 60

gtatcagctc actcaaaggc ggtaatacgg ttatccacag aatcagggga taacgcagga 120

aagaacatgt gagcaaaagg ccagcaaaag gccaggaacc gtaaaaaggc cgcgttgctg 180

gcgtttttcc ataggctccg cccccctgac gagcatcaca aaaatcgacg ctcaagtcag 240

aggtggcgaa acccgacagg actataaaga taccaggcgt ttccccctgg aagctccctc 300

gtgcgctctc ctgttccgac cctgccgctt accggatacc tgtccgcctt tctcccttcg 360

ggaagcgtgg cgctttctca tagctcacgc tgtaggtatc tcagttcggt gtaggtcgtt 420

cgctccaagc tgggctgtgt gcacgaaccc cccgttcagc ccgaccgctg cgccttatcc 480

ggtaactatc gtcttgagtc caacccggta agacacgact tatcgccact ggcagcagcc 540

actggtaaca ggattagcag agcgaggtat gtaggcggtg ctacagagtt cttgaagtgg 600

tggcctaact acggctacac tagaagaaca gtatttggta tctgcgctct gctgaagcca 660

gttaccttcg gaaaaagagt tggtagctct tgatccggca aacaaaccac cgctggtagc 720

ggtggttttt ttgtttgcaa gcagcagatt acgcgcagaa aaaaaggatc tcaagaagat 780

cctttgatct tttctacggg gtctgacgct cagtggaacg aaaactcacg ttaagggatt 840

ttggtcatga gattatcaaa aaggatcttc acctagatcc ttttaaatta aaaatgaagt 900

tttaaatcaa tctaaagtat atatgagtaa acttggtgaa ttcgcaaggt atagcccaat 960

atactttact tcgtaaagtt tgagctctgc gaggcaaaaa cgtcagatat tgaatctaaa 1020

caagagtata ataaacacag ttacacaaat aaaaacaaaa ggagtagatc ttaaaaatgg 1080

aagcaaatcc aaagtggcaa gaaacaggaa gacaacaaaa agtaaaacaa cttgactatt 1140

cagaaacatt agcagtaaga atagacaatg tggaattata caactcagaa aagaaattat 1200

attatctagt aattcaaacc cttcttggga atccccaaaa aaatgaatgg cgtatccagg 1260

gacaaagtca aatattaaaa gaatcaaaac aaaaaataat tgtgtcataa aattattgca 1320

cattaaaagt cctaaaaatt attttgcatt aaagatttca aaactcattt aatgtaaaat 1380

aattttttct ttgataatcc catttagcaa caatctcgcg taccacttgg tcaacctttt 1440

gatcatcact atccgtatta atcaaatcac cattctcaac gtcttctaat tgcaactgct 1500

tgcgaatttc tttcagcaaa ccgccatagc taatttgccg ggaaccagcc aaagctcgtt 1560

ccaaatcatc aattacttgt aggtcttgtt cttgattatt agtcaaaata tctttagatt 1620

ttacctgata tttagccgtt tcttgagcac tagccaataa cgaattttta tggcgattca 1680

tattcggttt aactgcctca acattcacca ttggtacata agtcaatttc attgcccgtt 1740

gccaaaaacc agtccattct acttgtgaaa tatagttatc agtcccctta aaataatggt 1800

tcttcacaaa aagcaaaaca tgcatatgat ggtggtacat tggctgaccc gcttcatgat 1860

taacagtcac ttcagtcgaa cgtacatagc ccaataaatt tttggcaact tttgtatact 1920

gaaaaatttt tgcaacagct cgtcccattt gacgtaactc actcttcaat tgatcaccag 1980

ttgtattctc aaccgtcaac gttaaaaata agaaccggcc cgtctttcgt tgtttaacag 2040

cttccgtcaa aatctgtgtt aactgattgg attgcttcat tgcccgccgc caattacata 2100

acggacacaa acgagaatgg caaaaccaag tctgcgccaa tttcttgtga ccatttttat 2160

cttccacaaa acgcaaaact tcgccacatt ctttaactcg atgagctttc ttataatgca 2220

atatttgtaa atagtcacca tactgcaagt tctccaactt gcgctcccgc cacggccgaa 2280

ctttccctga ctttgaccga tcaaccaaga ctttttcatt agccaaaata aaaactcccc 2340

tcaccaacca cgtgagaaga gttaatctgt tatgtacttg cttcacttaa atcagtcaga 2400

aggcttgacg gcaaagggtt caagctttaa actatgtcta gtaaatccaa atacgatttt 2460

tacaagatta actcacgcgt tcgaggtcgg caaactttcg aagctcacgt gggttttttt 2520

tatatttatt ttataccaca ataatacgcc taaacccagt tgtgtcaagg gtttacctca 2580

ctttttgaaa atgacgttgt ttctaatagt atcaagataa gaagaaaccg tcgaaaaaac 2640

gacggtttca aaccccaaaa agcagagaat tcggtacctg gagctgtaat ataaaaacct 2700

tcttcaacta acggggcagg ttagtgacat tagaaaaccg actgtaaaaa gtacagtcgg 2760

cattatctca tattataaaa gccagtcatt aggcctatct gacaattcct gaatagagtt 2820

cataaacaat cctgcatgat aaccatcaca aacagaatga tgtacctgta aagatagcgg 2880

taaatatatt gaattacctt tattaatgaa ttttcctgct gtaataatgg gtagaaggta 2940

attactatta ttattgatat ttaagttaaa cccagtaaat gaagtccatg gaataataga 3000

aagagaaaaa gcattttcag gtataggtgt tttgggaaac aatttccccg aaccattata 3060

tttctctaca tcagaaaggt ataaatcata aaactctttg aagtcattct ttacaggagt 3120

ccaaatacca gagaatgttt tagatacacc atcaaaaatt gtataaagtg gctctaactt 3180

atcccaataa cctaactctc cgtcgctatt gtaaccagtt ctaaaagctg tatttgagtt 3240

tatcaccctt gtcactaaga aaataaatgc agggtaaaat ttatatcctt cttgttttat 3300

gtttcggtat aaaacactaa tatcaatttc tgtggttata ctaaaagtcg tttgttggtt 3360

caaataatga ttaaatatct cttttctctt ccaattgtct aaatcaattt tattaaagtt 3420

catttgatat gcctcctaaa tttttatcta aagtgaattt aggaggctta cttgtctgct 3480

ttcttcatta gaatcaatcc ttttttaaaa gtcaatatta ctgtaacata aatatatatt 3540

ttaaaaatat cccactttat ccaattttcg tttgttgaac taatgggtgc tttagttgaa 3600

gaataaagac cacattaaaa aatgtggtct aagcttctgc aggatatccg atcgtccaca 3660

atcaaggtgc ttggcttttt cgatcgcgag gtcaccatgt acatcagtcg tgagagcatt 3720

gtgttgacag tgatcggcat cgtgttcggc tatctgctcg gcaatttgct gacagcctac 3780

attttgtatc aagccgaaac tgaggccgtg gtttttccac tcacgatcag cattgtcggc 3840

tacctcacgg ccacgttact catgttggcc ttcaccggcg tcgtcacctg gctcacgcat 3900

cgtcgactcc aacgggtgga catggtcgaa gccctgaaat caaacgaata acctacaatt 3960

ttgtcaggca gcgtcgtcac ggcgctgctt ttttcataca aaattcatca aaaattggga 4020

ttaaaaacgt tcatgatcgc aattttgaag cgcaaatgaa gattgagacc aactcctaac 4080

agtcctgtaa cgctgacgta acattgacac agtaaagtag cctttagtta atcaaattaa 4140

gggtgaggtc aaaaatgaaa ttcaataaag tcatgatcac gttggttgct gcagttacct 4200

tagcaggttc tgctagcgcc gtaacaccag ttttcgctga tacaagcgtc gacctcgag 4259

<210> 14
<211> 84
<212> DNA
<213> Artificial

<220>
<223>  VIP variant 1

<400>  14
cattcagatg ctgttttcac cgataactat acccgtttgc gtcgtcaatt ggctgttcgt        60

cgttatttga actcaatctt gaac                                               84